# EUROPEAN PATENT APPLICATION

(11) **EP 2 228 652 A1**
(43) Date of publication of application: **15.09.2010**
(21) Application number: 09154836.2
(22) Date of filing: 11.03.2009
(51) Int. Cl.: G01N 33/52

(54) **Improvement of tumour treatment**

(71) Applicant: Medizinische Universität Wien, 1090 Vienna (AT)
(72) Inventor: Krainer, Michael, 1190 Vienna (AT); Wittinger, Michael, 1160 Vienna (AT)
(74) Representative: Sonn & Partner Patentanwälte

(57) **Abstract**

The patent discloses the use of proteins of the endosomal sorting complex required for transport (ESCRT), especially of Hepatocellular Carcinoma Related Protein 1 (HCRP1) as a marker for the efficiency of an anti-EGFR antibody treatment of a tumour patient. If a tumour biopsy sample of the tumour patient reveals an ESCRT protein or mRNA amount equal to or above the amount in normal, non-tumour tissue, then the tumour patient is eligible for the treatment with an anti-EGFR antibody.

## Description

The present invention relates to improvement of tumour treatment.

Overexpression of epidermal growth factor (EGF) ligands and receptors (EGFR) has been implicated in promoting the hallmark of neoplastic traits of mitogenesis, inhibition of apoptosis, cell migration, metastases, angiogenesis and resistance to standard cytotoxic therapies.

The ERBB receptor network was extensively studied with respect to its signal transduction in recent years. The receptor family comprises four members (ERBB1-4), whereof ERBB2 and ERBB3 are non-autonomous functional. The dynamics between ERBB receptors and the molecules of the related signal cascades is under tight control at various levels. On the receptor level, this regulation includes alternative receptor dimerisation, receptor degradation and the recycling of the receptor back to the cell membrane. ERBB1 (EGFR) is a 170kDa membrane spanning protein that plays an essential role in regulating a number of cellular processes including cell proliferation, survival and migration. Activation by EGF or related signal molecules leads to a conformational switch that triggers dimerisation and autophosphorylation of defined tyrosine residues in the cytoplasmatic domain. The phosphorylated residues serve as binding sites for adaptor molecules, triggering the activation of different pathways like the RAS-RAF-MEK-ERK pathway, the PI3K-AKT pathway and the PLC-gamma-PKC pathway. Dysregulation of EGFR often comes along with tumour development and cancer patients with altered EGFR activity tend to have a more aggressive disease with a poor clinical outcome.

Experimental and clinical evidence indicates that EGFR inhibitors can simultaneously suppress many of these properties, leading to tumour stasis or regression. Several selective compounds that target either the EGFR extracellular ligand-binding region or the intracellular tyrosine kinase region are being developed. The most advanced of the newer therapies in clinical development and practice are anti-receptor monoclonal antibodies IMC-C225 (Cetuximab, Erbitux; Imclone), and the reversible small-molecule inhibitors of EGFR, ZD1839 (Gefitinib, Iressa; AstraZeneca) and OSI-774 (Erlotinib, Tarceva; OSI Pharmaceuticals).

In general, antibodies target EGFR by inhibiting ligand binding and receptor dimerisation, whereas small molecules competitively inhibit ATP binding to the receptor, thereby hindering autophosphorylation and kinase activation. Antibodies with intact Fc binding domains, such as Cetuximab, might also induce antibody-mediated cellular cytotoxicity (ADCC). Both classes of molecules induce dose-dependent tumour stasis or even tumour regression in some tumour xenograft models, and antiproliferative effects seem to be correlated with dose/concentration-dependent inhibition of EGFR phosphorylation. Preclinical models indicate that EGFR expression is required, although the degree of expression above an undefined threshold does not predict sensitivity to EGFR inhibitors. However, surprisingly little is understood about predictors of sensitivity to EGFR inhibitors, beyond the expression of EGFR and inhibition of EGFR phosphorylation (Dancey et al, Nature Reviews Drug Discovery 2, 296-313, 2003).

Small-molecule inhibitors seem to have different toxicity profiles and more variable pharmacokinetics than antibodies. Moreover, clinical trials indicate that such small molecules have plasma half-lives that allow once-daily dosing. However, inter-patient plasma concentrations at steady state and exposures can vary tenfold at a given dose, which reflects the variability in bioavailability and metabolism of these agents between patients.

It has been speculated that the EGFR family, rather than individual family members, should be regarded as the appropriate target for the development of cancer therapeutics. Molecules that inhibit several members of the EGFR family, or strategies that combine selective inhibitors of family members, might be more effective than inhibition of EGFR alone.

Anti-EGFR antibodies and small-molecule inhibitors of EGFR have different mechanistic and pharmacological properties, and are being evaluated in different clinical situations that might ultimately lead to appreciable differences in clinical outcomes. For example, the agents now under development or already authorised for marketing differ in their affinities for EGFR and other EGFR family members. Among the small molecules, GW2016 (a small molecule kinase inhibitor developed by GSK) inhibits EGFR and HER2, and CI1033 (a small molecule kinase inhibitor developed by Pfizer) inhibits EGFR, HER2 and HER4 with similar IC50 values. Antibodies such as Cetuximab with an intact Fc portion might also induce ADCC, which might prove to be therapeutically advantageous. The small molecules Gefitinib and Erlotinib have been evaluated at a biologically active dose with limited toxicity and at an MTD, respectively.

In WO 2007/050495 A2 a method for determining the likelihood of effectiveness of an EGFR targeting treatment in a subject affected with a tumour is described. This method comprises the detection of the presence or absence of EGFR expression in endothelial cells associated with the tumour, wherein the presence of EGFR expression indicates that the EGFR targeting treatment is likely to be effective. Under "EGFR targeting treatment", however, both, anti-EGFR-antibodies as well as small molecule inhibitors for EGFR are understood in WO 2007/050495 A2.

However, up to now, no test or marker exist which allow a reasonable treatment decision whether the treatment is performed with antibodies or small molecule inhibitors against EGFR or ERBB in general.

It is therefore an object of the present invention to provide a suitable marker which allows a reasonable decision for treatment of tumour patients whether the patient should receive EGFR antibodies (or ERBB antibodies) or whether the administration of small molecule inhibitors against EGFR (or ERBB in general) is the more preferred treatment for the individual patient. More specifically, it is an object of the present invention to provide tools which allow a reasonable decision whether the treatment with anti-EGFR antibodies can be effective at all (or whether such treatment cannot be expected to be effective in a given tumour patient).

The present invention therefore provides a method for determining the status of a tumour patient with respect to tumour treatment with an anti-Epidermal Growth Factor Receptor (EGFR-) antibody characterised by the following steps:
- determining the amount of mRNA or protein of endosomal sorting complex required for transport (ESCRT) selected from Hepatocellular Carcinoma Related Protein 1 (HCRP1), vacuolar protein sorting 23 (Vps23), vacuolar protein sorting 28 (Vps28) and multivesicular body sorting factor of 12 kD (Mvb12), in a tumour biopsy sample of a tumour patient;
- determining the status of the tumour patient as being eligible for the treatment with an anti-EGFR antibody if the ratio of the HCRP1, Vps23, Vps28 or Mvb12 amount as determined in the tumour biopsy sample and the HCRP1, Vps23, Vps28 or Mvb12 amount as determined in normal, non-tumour tissues is at least 0.8; or
- determining the status of the tumour patient as not being eligible for the treatment with an anti-EGFR antibody if the ratio of HCRP1, Vps23, Vps28 or Mvb12 amount determined in the tumour biopsy sample and the HCRP1, Vps23, Vps28 or Mvb12 amount in normal, non-tumour tissue is less than 0.8.

According to the present invention it has been found that ESCRT proteins and mRNA of HCRP1, Vps23, Vps28 or Mvb12 are suitable markers for assisting in the decision for the individually optimised treatment of tumour patients in tumour treatment involving EGFR inhibition. It was shown that a decreased expression of these ESCRT members in tumour tissue (for example ovarian cancer tissue) when compared to normal tissue is associated with a more aggressive disease.

The present invention is based on the use of HCRP1, Vps23, Vps28 or Mvb12 as biomarkers for the efficiency of an anti-EGFR antibody treatment of a tumour patient. If a tumour biopsy sample of the tumour patient reveals amounts being at least 0.8 (80%) or more of the HCRP1, Vps23, Vps28 or Mvb12 amount of a reference, then the tumour patient is eligible for the treatment with an anti-EGFR antibody. For the present invention, determination of one or more of these ESCRT members is possible for predicting the status of the tumour patient for anti-EGFR antibody treatment. Determination of HCRP1 protein or mRNA is preferred, especially in combination with one or more of Vps23, Vps28 and Mvb12 (mRNA or protein).

The present invention therefore provides a method for determining the status of a tumour patient with respect to tumour treatment with an anti-Epidermal Growth Factor Receptor (EGFR-) antibody characterised by the following steps:
- determining the amount of Hepatocellular Carcinoma Related Protein 1 (HCRP1) mRNA or protein in a tumour biopsy sample of a tumour patient;
- determining the status of the tumour patient as being eligible for the treatment with an anti-EGFR antibody if the ratio of the HCRP1 amount as determined in the tumour biopsy sample and the HCRP1 amount as determined in normal, non-tumour tissues is at least 0.8; or
- determining the status of the tumour patient as not being eligible for the treatment with an anti-EGFR antibody if the ratio of HCRP1 amount determined in the tumour biopsy sample is below the HCRP1 amount in normal, non-tumour tissue is less than 0.8.

According to the present invention it has been found that HCRP1 is a suitable marker for assisting in the decision for the individually optimised treatment of tumour patients in tumour treatment involving EGFR inhibition. It was shown that a decreased expression of HCRP1 in tumour tissue (for example ovarian cancer tissue) when compared to normal tissue is associated with a more aggressive disease. In vitro, it was demonstrated that HCRP1 is involved in the downregulation process of activated EGFR and HER2 in ovarian cancer cell lines (SK-OV-3, MDAH). Moreover, its downregulation counteracts the inhibitory effect of EGFR or ERBB antibodies, such as Cetuximab, but not of small molecule inhibitors, such as Lapatinib.

The present invention is based on the use of HCRP1 as a bio-marker for the efficiency of an anti-EGFR antibody treatment of a tumour patient. If a tumour biopsy sample of the tumour patient reveals HCRP1 amounts being at least 0.8 (80%) or more of the HCRP1 amount of a reference, then the tumour patient is eligible for the treatment with an anti-EGFR antibody.

As reference for normal, non-tumor tissue may be a non-tumor section of the sample tissue taken from the same patient. A further reference may be the experimentally determined tissue specific expression of HCRP1. A further reference may be the expression levels as identified in a number of normal tissues. A further reference may be an absolute reference.

The reference is exemplarily defined by one of the following procedures. 1) Determination of HCRP1 mRNA or protein expression levels in the normal tissue and the cancerous tissue of the same patient, e.g. using a tissue biopsy. If the ratio between expressions in cancerous versus normal tissue is at least 0.8, or equal, or greater than one, treatment with anti-EGFR antibodies in indicated. 2) HCRP1 mRNA and proportionally protein expression levels published in the Body Map GEO dataset GSE7905 (Barrett et al., Nucleic Acids Research, 2005, Vol. 33, Database issue D562-D566) are used as reference. If the HCRP1 mRNA or protein expression is at least 80%, equal or above this reference, treatment with anti-EGFR antibodies is indicated. 3) HCRP1 mRNA or protein expression levels as identified in a set of normal tissues are used as absolute reference. If the HCRP1 mRNA or protein expression is at least 80%, equal or above this reference, treatment with anti-EGFR antibodies is indicated. 4) Hybridization to a defined number of HCRP1 primer molecules or anti-HCRP1 antibody binding to a defined number of HCRP1 antigens is used as absolute reference. If the HCRP1 mRNA or protein expression is at least 80%, equal or above this reference, treatment with anti-EGFR antibodies is indicated. Preferably, the ratio is defined as being higher than 0.8, for example 1, especially more than 1.

There is empiric evidence that dysfunctional overexpression of the EGFR is associated with unfavourable prognosis particularly in cases of low HCRP1 levels. This may be explained by cytoplasmic accumulation of activated receptors (pEGFR) secondary to a deficient degradation process. According to the aforementioned exclusion criteria for therapies with monoclonal antibodies these patients should be treated with small molecule inhibitors targeting the EGFR.

Aside from defects of the degradation process, EGFR hyperactivation can be driven by different means like activating mutation, enhanced ligand expression, or gene amplifications, potentially leading to high amounts of active receptors independently of the HCRP1 status. Consequently, tumours with physiological or high HCRP1 expression as well as high pEGFR amounts should be treated with monoclonal antibodies like Cetuximab. Three scenarios with the corresponding treatment options can be distinguished: 1) low HCRP1 and consequently high pEGFR amounts indicates treatment with small molecule inhibitors; 2) high HCRP1 and high pEGFR amounts indicates treatment with monoclonal antibodies against EGFR; 3) high HCRP1 and low pEGFR amounts indicates no therapy targeting the EGFR. This procedure can be simplified by incorporating the results of an immunofluorescence experiment, which shows if the activated EGFR accumulates within the cytoplasm as a consequence of deficient receptor degradation. Accordingly, determining the quantity and location of pEGFR is a surrogate for HCRP1 in the decision between antibody- or small molecule based therapies targeting the EGFR or other RTKs.

HCRP1 was described as a tumor suppressor-associated gene. The HCRP1 gene localizes in 8p22 region of the human chromosome, where loss of heterozygosity (LOH) occurs highly frequent in many tumors. Full-length sequencing showed that HCRP1 cDNA is 1917bp in length, which comprises a complete open reading frame (nucleotides 151-1341) encoding a 397aa tumor suppressor protein named HCRP1 (US 2006/116323 A). It was shown that HCRP1 is significantly reduced or undetected in hepatocellular carcinoma (HCC). Its overexpression in HCC cell line SMMC-7721 significantly inhibited anchorage-dependent as well as anchorage independent cell growth in vitro, whereas the knock down of HCRP1 in HCC cell line BEL-7404 resulted in enhanced cell growth (Xu et al., Biochemical and Biophysical Research Communications 311, 1057-1066, 2003). Depletion of HCRP1 diminishes epidermal growth factor receptor (EGFR) degradation in the HeLa cell line.

There are currently two main categories of targeted therapies against tumours with enhanced EGFR signalling, namely monoclonal anti-EGFR antibodies and small molecule EGFR tyrosine kinase inhibitors (TKIs).

In contrast to an antibody a small molecule is a term used commonly in pharmacology to denote a small organic compound that is biologically active (biomolecule) but is not a polymer. This term is very loosely used and it may or may not include monomers or primary metabolites, in fact it is generally used to denote molecules that are not proteins which play an endogenous or exogenous biological role, such as cell signalling, are used as a tool in molecular biology or are a drug in medicine. These compounds can be natural (such as secondary metabolites) or artificial (such as antiviral drugs); they may have a beneficial effect against a disease (such as FDA approved drugs) or may be detrimental (such as teratogens and carcinogens). Preferred molecular weights of small molecule EGFR inhibitors according to the present invention are in the range of 200 to 2000 Da, preferably 300 to 1500 Da, especially 400 to 1000 Da.

Preferred small molecule EGFR inhibitors according to the present invention are Gefitinib, Erlotinib, AEE788, CI-1033, HKI-272, HKI-357 or EKB-569.

A number of small molecule inhibitors of EGFR are disclosed in paragraphs [00124] to [00126] of WO 2007/050495 A2. Further useful EGFR inhibitors are described in U.S. Pat. App. No. 20040127470, particularly in tables 10, 11, and 12.

Some inhibitors of ErbB2 also inhibit EGFR and may be useful in the methods of the present invention. ErbB2 inhibitors include CI-1003, CP-724,714, CP-654577 (Pfizer, Inc.), GW-2016, GW-282974, and lapatinib/ GW-572016 (Glaxo Wellcome pic), TAK-165 (Takeda), AEE788 (Novartis), EKB-569, HKI-272 and HKI-357 (Wyeth) (Wyeth-Ayerst) and EXEL 7647/EXEL 0999 (EXELIXIS).

On the other hand, if the tumor treatment according to the present invention is eligible to be a treatment with an EGFR antibody, examples of such antibodies are described e.g. in paragraphs [00127] to [00128] of WO 2007/050495 A2. For example, antibodies which interfere with kinase signaling via EGFR, including monoclonal, chimeric, humanized, recombinant antibodies and fragment thereof which are characterized by their ability to inhibit the kinase activity of the EGFR and which have low toxicity.

Neutralizing antibodies against EGFR are readily raised in animals such as rabbits or mice by immunization with an EGFR. Immunized mice are particularly useful for providing sources of B cells for the manufacture of hybridomas, which in turn are cultured to produce large quantities of anti-EGFR monoclonal antibodies. Chimeric antibodies are immunoglobin molecules characterized by two or more segments or portions derived from different animal species. Generally, the variable region of the chimeric antibody is derived from a non-human mammalian antibody, such as murine monoclonal antibody, and the immunoglobin constant region is derived from a human immunoglobin molecule. Preferably, both regions and the combination have low immunogenicity as routinely determined. Humanized antibodies are immunoglobin molecules created by genetic engineering techniques in which the murine constant regions are replaced with human counterparts while retaining the murine antigen binding regions. The resulting mouse- human chimeric antibody should have reduced immunogenicity and improved pharmacokinetics in humans. Examples of high affinity monoclonal antibodies and chimeric derivatives thereof, that are useful in the methods of the present invention, are described in EP 0 186 833 A, WO 92/16553 and US 6,090,923. Suitable clinical antibodies are described in Reichert et al., Nature Reviews Drug Discovery 6, 349-356 2007).

Suitable anti-EGFR antibodies include the monoclonal antibodies Cetuximab, Panitumumab, Matuzumab (Merck KGaA) and anti-EGFR 22Mab (ImClone Systems Incorporated of New York, New York, USA), or egf/r3 MAb (Cuban Institute of Oncology), Nimotuzumab ((TheraCIM-hR3) YM BioSciences Inc. Mississauga, Ontario, Canada), EMD-700, EMD-7200, EMD-5590 (Merck KgaA), E7.6.3 (Abgenix), Mab 806 (Ludwig Institute), MDX-103, MDX-447/H-477 (Medarex Inc. of Annandale, N.J., USA and Merck KgaA), and the monoclonal antibodies Trastuzumab (tradename HERCEPTIN), 2C4 (Genentech), AR-209 (Aronex Pharmaceuticals Inc. of The Woodlands, Tex., USA), Pertuzumab (tradename OMNITARG; Genentech), BMS-599626 (Bristol-Myers Squibb) and 2B-1 (Chiron).

Cetuximab (Erbitux; Imclone Systems Inc., New York, NY; Nat.Rev.Drug Disc. 3 (2004), 549-550), a monoclonal antagonistic antibody against EGFR (and used for colorectal cancer), induces dimerisation, internalisation and subsequent degradation of the receptor. The alternative pathway of recycling the receptor back to the cell membrane is inhibited, since Cetuximab does not dissociate from the receptor within the endosome, which is the case for native ligands as EGF. Cetuximab can inhibit downstream signaling by preventing autophosphorylation and activation of the receptor. In contrast, it is also reported that Cetuximab transiently stimulates EGF phosphorylation prior to downregulation.

Panitumumab (Amgen, Thousand Oaks, California; Nat.Rev.Drug Disc. 5 (2006), 987-988) is an alternative therapeutic antibody targeting the EGFR (for the treatment of colon and rectum carcinoma) and further antibodies including Matuzumab (Merck KgaA and EMD Pharmaceutical, Durham, North Carolina), Zalutumumab (Genmab, Kopenhagen, Dänemark), Nimotuzumab (YM BioSciences Inc., Mississauga, Canada), or Pertuzumab (Genentech, San Francisco, California) are currently tested in clinical trials or are still under development.

According to preferred embodiments of the present invention, the anti-EGFR antibody is in particular Cetuximab, Matuzumab, Pertuzumab, Zalutumumab, Nimotuzumab or Panitumumab. As stated above, these antibodies have already proven clinical efficacy in tumour treatment.

The tumour patient may be a tumour patient with a solid tumour and is preferably a tumour patient having gastrointestinal cancer, prostate cancer, ovarian cancer, breast cancer, head and neck cancer, lung cancer, non-small cell lung cancer, cancer of the nervous system, kidney cancer, retinal cancer, skin cancer, stomach cancer, liver cancer, pancreatic cancer, genital-urinary cancer, prostate cancer, colorectal cancer, rectal cancer or bladder cancer. Patients with ovarian cancer are specifically preferred. These tumours are known to be potentially responsive for anti-EGFR-treatments (WO 2007/050495 A2).

The present invention also relates to a method for determining the likelihood of effectiveness of an anti-EGFR antibody treatment in a tumor patient which comprises determining the amount of Hepatocellular Carcinoma Related Protein 1 (HCRP1) in a biopsy sample of a tumour patient and determining the likelihood of effectiveness of an EGFR targeting treatment with an anti-EGFR antibody if the HCRP1 amount determined in the tumour biopsy sample is at least 80% of the HCRP1 amount in normal, non-tumour tissue.

The present invention further relates to a method of treating a patient affected with or at risk for developing a tumour with an anti-EGFR antibody, comprising detecting the presence or absence of reduced HCRP1 expression in a tumour biopsy of said patient, wherein the patient is administered an anti-EGFR antibody treatment if the absence of the reduced HCRP1 expression is detected.

The present invention also relates to a method to direct treatment of a patient with a tumor, wherein the HCRP1 expression status of a tumour biopsy of the patient, and here the absence of a reduced HCRP1 expression directs treatment of the patient points towards administration of an anti-EGFR antibody treatment.

According to another aspect, the present invention relates to the use of a kit for determining the expression of HCRP1 in a tumour biopsy sample, comprising
- an antibody against HCRP1, and
- detection means for the antibody against HCRP1;
for predicting the efficiency of an anti-EGFR antibody treatment of the tumour patient.

Alternatively, the present invention relates to the use of a kit for determining the expression of HCRP1 in a tumour biopsy sample, comprising
- a primer against HCRP1, and
- detection means for the amount of mRNA of HCRP1 as determining by quantitative RT-PCR
for predicting the efficiency of an anti-EGFR antibody treatment of the tumour patient.

Techniques for the determination of the expression of HCRP1, including suitable antibodies against HCRP1, are readily available for the person skilled in the art and can e.g. be derived from Xu et al., 2003, US 2006/0116323 A1, etc.. Techniques in particular include quantitative RT-PCR for measurement of HCRP1 mRNA in tumor tissue or immunohistochemistry using an anti-HCRP1 antibody, or FACS analysis using an anti-HCRP1 antibody. Embodiments for determining HCRP1 expression which are specifically preferred according to the present invention is the determination on mRNA level by quantitative RT-PCR, or on protein level by Immunohistochemistry (IHC).

According to another aspect, the present invention relates to an antibody against HCRP1 **characterised in that** it is raised against an antigenic determinant of HCRP1 selected from the sequence MSPYASGGFPFLPPY, WLFPLTKSASSSAAG or TSHTTAKPAAPSFGV. Antibodies against these antigenic determinants should significantly improved characteristics, especially for detecting HCRP1 in tumour biopsy samples but also in effectively raising poly- and monoclonal antibodies thereto. Raising antibodies with one or more of there three epitopes, in particular with one of the three linear epitopes given as amino acid sequence 3 - 17 (WLFPLTKSASSSAAG), amino acid sequence 147 - 161 (MSPYASGGFPFLPPY), and amino acid sequence 187 - 198 (TSHTTAKPAAPSFGV) of HCRP1 is specifically useful for the present invention. For the present invention an antibody against MSPYASGGFPFLPPY was used exemplarily.

The present invention is further illustrated by way of the following examples and figures.

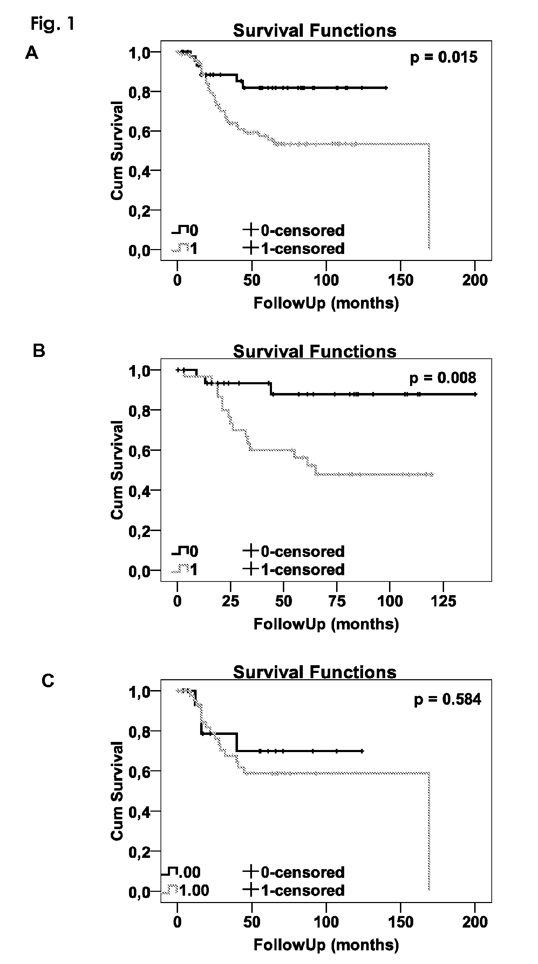

Fig. 1: Tissue microarrays composed of 125 primary ovarian tumor samples and 19 borderline tumors spotted in triplicates and corresponding normal tissue was stained for HCRP1 and EGFR. Staining intensities are classified as described in Material and Methods. Statistical analysis was realized by Cox proportional hazards regression models for survival analysis, depicted in plots of the corresponding Kaplan-Meier estimations. Fig. 1A shows the prognostic significance of EGFR on overall survival. Fig. 1B/C illustrate the modulation of EGFR prognostic value by low (1B) or high (1C) HCRP1 expression.

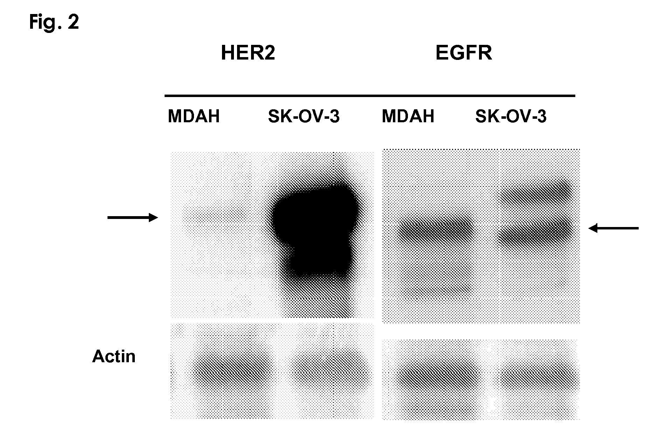

Fig. 2 shows that EGFR is equally expressed in MDAH-2774 and SKOV-3 cell lines, whereas HER2 is almost undetected in MDAH-2774 and overexpressed in SKOV-3 as determined by Western blotting. This setup of receptor levels is a basis for studying the dynamics between HCRP1, EGFR and HER2 in these cell lines;

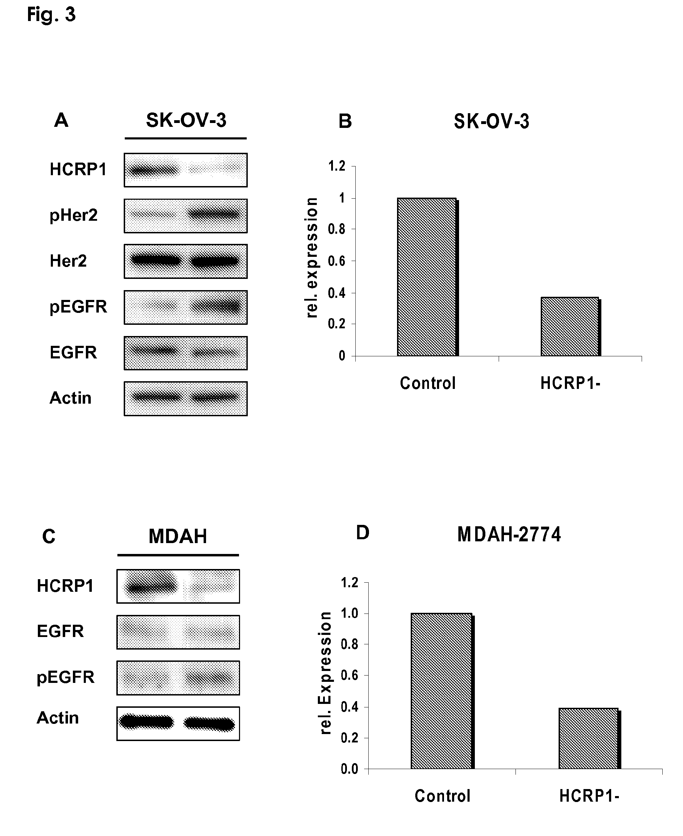

Fig. 3 shows that knock-down of HCRP1 (confirmed on mRNA level, Fig.2A and 2C) leads to increased amounts of pEGFR in both SK-OV-3 and MDAH-2774 cell lines as determined by Western blotting (Fig. 2B and 2D). In the HER2 positive SKOV-3 cell line pHER2 levels were also found to be enhanced;

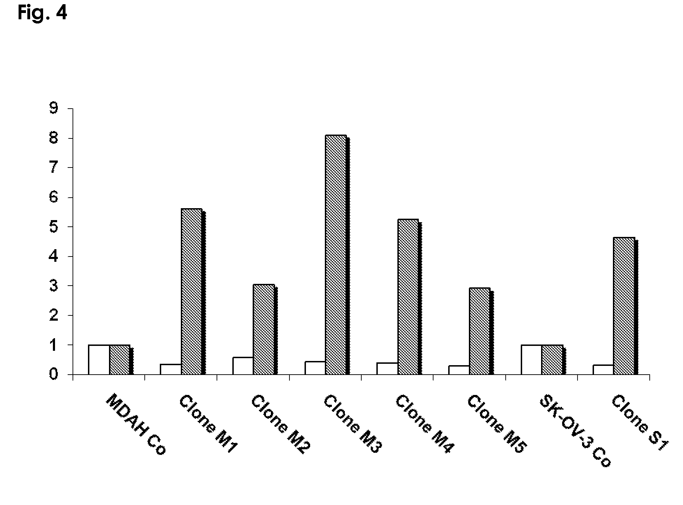

Fig. 4 shows that the inverse correlation between HCRP1 expression (white panel) and the ratio between activated and total EGF receptor (grey panel) is stable over several clones silenced for HCRP1;

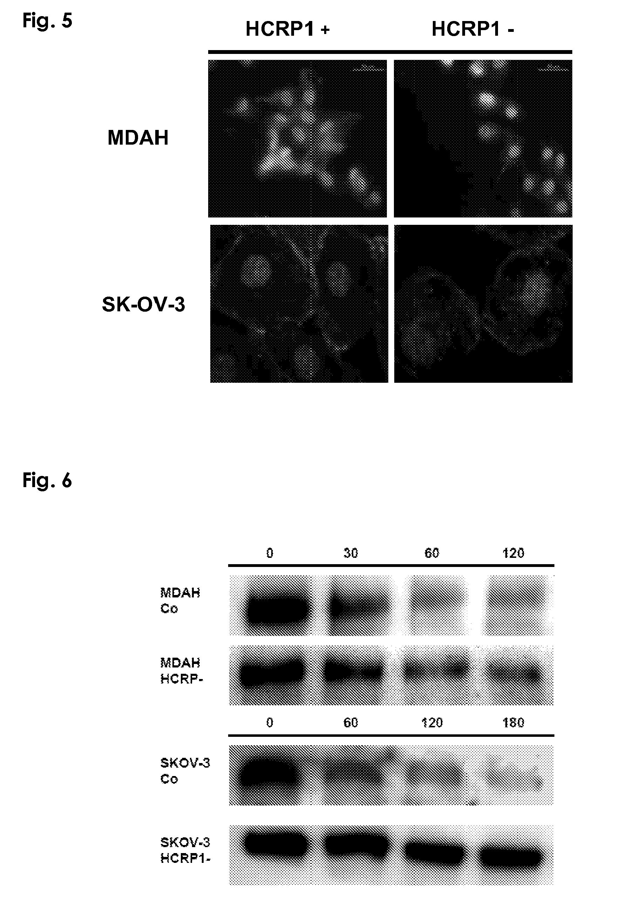

Fig. 5 shows the cytoplasmic accumulation of the activated EGFR in HCRP1-silenced MDAH and SK-OV-3 cell lines as determined by immunofluorescent staining of pEGFR.
Fig. 6 shows cells starved for 24 hours and subsequently stimulated with 20ng/ml EGF, followed by harvesting of the proteins at defined points in time for determining EGFR levels by Western blotting. The degradation efficiency was observed to be dramatically inhibited in the silenced cell lines leading to accumulation of pEGF;

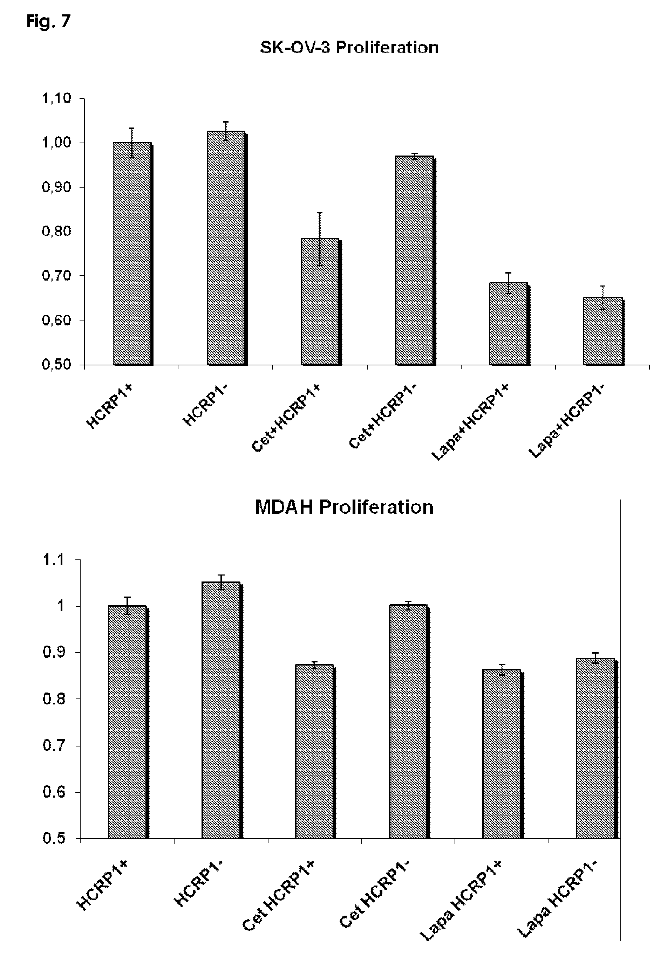

Fig. 7 shows that proliferation of MDAH-2774 and SKOV-3 cell lines was not (or only slightly) affected by HCRP1 knock-down. However, the anti-proliferative effect of Cetuximab was observed only in the controls but was reversed in the HCRP1-silenced cell lines. In contrast, the inhibitory effect of Lapatinib was independent of HCRP1 expression; and

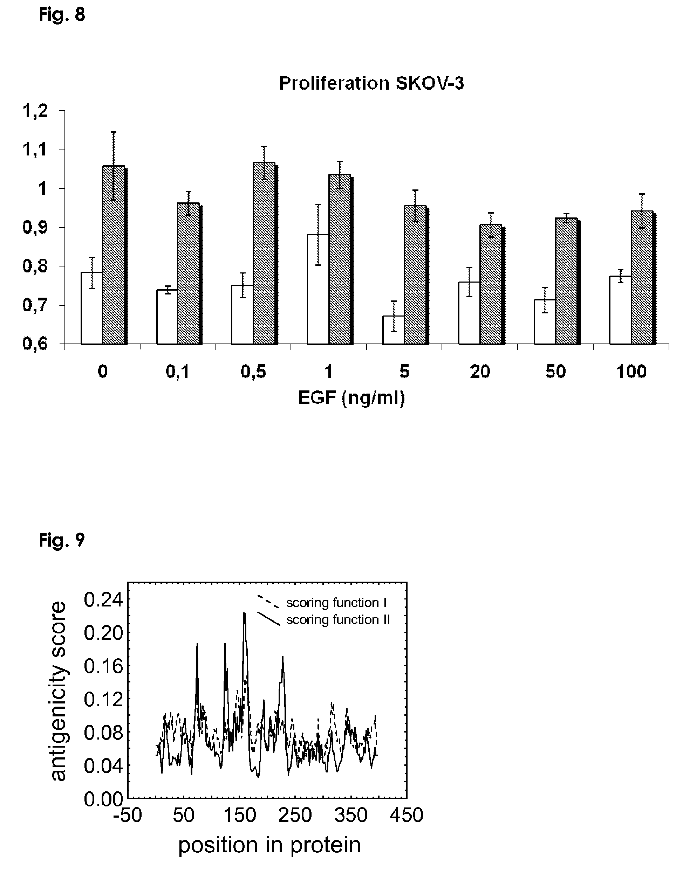

Fig. 8 confirms the effect of HCRP1 on Cetuximab-treated SKOV3 cells by using a CellTiter-Blue assay as a second, independent method. The impact of HCRP1 expression on the proliferation was calculated as quotient of HCRP1+ and HCRP1- cells. A ratio of 1 would mean that no change of proliferation was detected between HCRP1+ and HCRP1- cells. Ratios below/above 1 come along with increased/decreased proliferation of HCRP1+ cells or vice versa for HCRP1- cells. HCRP1 knock down yielded no effect on proliferation in non-stimulated cells (grey panels), whereas EGF concentrations of 5ng/ml (as indicated on the x-axis) or higher resulted in a slightly increased proliferation of HCRP1- cells (ratio=0.8-0.9). Upon incubation with Cetuximab (white panels) the ratio declined significantly (0.68-0.78). This finding can be traced back to decreased proliferation of HCRP1+ cells, while HCRP1- cells were not affected.
Fig. 9 shows a proprietary bioinformatics antigenicity scoring of HCRP1. Two antigenicity scores were applied on the primary protein sequence (scoring function 1, dashed line, and scoring function 2, solid line, where high values indicate high, low values low antigenicity) are plotted versus the amino acids sequence of HCRP1 from N- to C-terminus. In particular three peaks indicating high antigenicity are identified when combining both scores. A linear peptide covering a high scoring region was used for immunization for generating an anti-HCRP1 antibody.

### EXAMPLES:

In the study according to the present invention it is shown that the expression of HCRP1 is lower in ovarian cancer tissue than in regular tissue and that this downregulation is associated with a more aggressive disease, particularly in certain patient subgroups. In vitro, it could be demonstrated that HCRP1 is involved in the downregulation process of activated EGFR and HER2 in ovarian cancer cell lines (SK-OV-3, MDAH). Moreover, its downregulation overcomes the inhibitory effect of Cetuximab but not of Lapatinib.

This could be due to the following mechanism: Certain tumours, to increase their selective advantage in the course of their development, downregulate HCRP1 to upregulate the number of functional EGF receptors. Tumours with upregulated EGFR should be prone to a disruption of this signaling axis. But in contrast to the inhibition by small molecules, the inhibition by EGFR antibodies does not work in a HCRP1 negative background, since in absence of HCRP1 the degradation and consequently inactivation of the EGFR/antibody complex is not possible. The in vitro data obtained and described below are in line with this proposed mechanism.

### Patient Material

The patient material for the tissue microarray (TMA) was composed of 125 primary ovarian tumor samples and 19 borderline tumors spotted in triplicates and corresponding normal tissue. None of the patients with borderline tumors died during the follow-up time and were excluded from the survival analysis.

### Data analysis and statistics

In order to compare HCRP1 expression between primary tumors, recurrent tumors and normal controls a Mann-Whitney U test was performed. The potential influence of HCRP1 expression on overall survival is presented in plots of the corresponding Kaplan-Meier estimates and quantified using Cox regression first with a univariate model and then adjusted for grading, staging according to FIGO and morphologic subtype of the tumor samples. 95% confidence intervals were calculated. HCRP1 was dichotomized at the median. Histology was dichotomized into serous and non-serous tumors. Tumor stage and grade were used as continuous variables. P-values ≤0.05 were considered to be statistically significant.

In the tissue microarray staining intensities were analyzed by two independent persons and classified in 0 (missing expression), 1 (low expression), 2 (medium expression) and 3 (high expression). Equal to the mRNA expression analysis experiment, HCRP1 was dichotimized into two groups at the median. For EGFR, results of triplicates and both interpretations were averaged and re-scaled (0-3). Since 40% of the tumor samples stained negatively or very weakly positively for EGR, we divided into two groups of positive and negative EGFR expression. Kaplan-Meier plots and survival analysis were calculated as described above. All statistical analyses were performed on SPSS 15 (Chicago, IL, USA).

### Tissue Microarray staining procedure

After deparaffinization and rehydration the samples were treated with 0.3% H2O2/PBS (pH 7.4) for 10 minutes to quench endogenous peroxidase activity and blocked with serum of the secondary antibody diluted 1:50 in PBS. Primary antibodies against EGFR produced in rabbit and HCRP1 were diluted 1:100 in serum/PBS and applied on the samples for 1 hour. The secondary antibody against rabbit was applied for 30 minutes. After visualization with DAB+ (Dako, CA, USA) and counterstaining with hematoxyline/eosin the slides were mounted in Eukitt (O. Kindler GmbH, Freiburg, Germany) and analyzed on an Olympus BX50 upright light microscope (Olympus Europe, Hamburg, Germany) equipped with the Soft Imaging system CC12.

Each sample was treated in an identical manner and the entire cohort was analyzed in one batch on three slides. Reagent conditions, incubation times and temperatures and antigen retrieval (if necessary) were held identical for each case as previously described.

### Knock-down of HCRP1 in ovarian cancer cell lines

The SK-OV-3 cell line was cultured equally the MDAH-2774 cell line except for the use of McCoy's medium. For applying the Tet-Off inducible system, founder cell lines were generated by transfecting SK-OV-3 cells with the pTet-Off vector (neor) encoding a tetracyclin repressible transactivator (tTA). Resistant colonies were selected with 200 µg/ml G418 and characterized by transient transfection with the luciferase reporter plasmid (pTRE-Luc), whose promoter is induced by the transactivator. Luciferase activity in the presence and absence of tetracycline was measured to identify cells with maximal promoter inducibility. Highest induction was 92-fold. These two founder cell lines were used to establish cells inducible for HCRP1-specific shRNA constructs (Open Biosystems; #V2HS_21202, #V2HS_21203), which were cloned into the SIN-TREmiR30-PIG vector (purr) downstream of the tetracycline inducible promoter. Thus, presence of tetracycline in the culture medium would suppress shRNA expression, while its withdrawal would induce the knock-down of HCRP1. Puromycin-resistant colonies were isolated in the presence of tetracycline, and screened for silencing efficiency of HCRP1 by qRT-PCR and Western blotting.

The human ovarian carcinoma cell line MDAH-2774 was cultured in RPMI medium with 10% FCS (fetal calf serum), 50 units/ml penicillin G, and 50 µg/ml streptomycin sulfate at 37°C in a humidified atmosphere of 95% air with 5% CO₂. three different shRNA constructs complementary exclusively to the HCRP1 mRNA in addition to 1 nonsense construct serving a control were cloned into the vector pSilencer 4.1-CMV neo. Transfection was performed with Lipofectamine 2000 according to the manufacturers protocol (Invitrogen, Carlsbad, CA, USA). Stable clones were selected with 700 µg/ml G418, picked and sub-cultured with 350 µg/ml G418. Silencing efficiency was quantified by qRT-PCR and western blot.

### Antibody Production

A polyclonal peptide antibody against HCRP1 was established. The following peptide sequence was selected following a bioinformatics analysis of the HCRP1 protein sequence: MSPYASQGFPFLPPY. This sequence indicated highly increased antigenicity when compared to other sequence sections of HCRP1 with proprietary analysis tools (Fig. 9). Rabbit antiserum raised against this peptide sequence was prepared by Eurogentec (Seraing, Belgium). The serum was affinity purified on beads containing the selected peptide and tested for its applicability in Western blotting and immunohistochemistry.

### Protein Preparation and Western Blotting:

The following antibodies were used in the dilution indicated: HCRP1 1:200 (established as described above); primary antibodies ( EGFR 1:300, pEGFR 1:100, Her2 1:200, pHer2 1:200 and beta-actin 1:300) and HRP-conjugated secondary antibodies (anti-goat 1:10000 and anti-rabbit 1:10000) were obtained from Santa Cruz Biotechnology Inc. (Santa Cruz, CA, USA). Upon treatment, cells were lysed and protein was prepared with RIPA+ buffer, protein concentration was determined by a standard Bradford absorbance assay (Sigmal Aldrich, Saint Louis, MO, USA). Equal amounts of proteins (30µg) were separated by SDS-PAGE, blotted on PVDF membranes (GE Healthcare, Buckinghamshire, UK), incubated with the appropriate primary antibody and visualized via HRP-conjugated secondary antibodies and treatment with the ECL chemiluminescent detection system (GE Healthcare, Buckinghamshire, UK).

### Fluorescence Microscopy

Cells were seeded on chamber slides, grown for 48 hours, fixed with 4% paraformaldehyde for 15 minutes and permeabilized with 0.1% Triton X-100. The subsequent staining procedure was performed as described for immunohistochemistry. The cells were incubated with the primary antibody (goat) directed against pEGFR (1:100) for 1 hour. TRITC-conjugated secondary antibody (1:100) was applied on the cells for 45 minutes and counterstaining of the nuclei was realized by DAPI (Roche, Manchester, England) or Quinacrine. Afterwards the cells were analyzed on a fluorescence microscope (Nikon Eclipse 800) equipped with a Nikon DS-R1 camera by using the NIS-Elements software.

### Proliferation Assays

2x10⁵ SK-OV-3 (with and without doxycyclin) and MDAH (HCRP1 silenced, nonsense control and wildtype) cells were seeded in 6-well plates in media complemented with 10% FCS and treated with 20 µg/ml Cetuximab (Merck, Whitehouse Station, NJ, USA), 4 µM Lapatinib (GlaxoSmithKline plc. London, UK) or were mocktreated. To maintain the logarithmic phase, cells were split at intervals of 48 hours, along with the determination of the cell number by a CASY cell counter (Innovatis AG, Bielefeld, Germany). This procedure was performed in triplicates over a timespan of 6 days and confirmed by a replication of the whole experiment. From the obtained data doubling times (dt) were calculated and depicted as dt-1.

### RESULTS:

### Prognostic value of EGFR and Her2 is tightly related to HCRP1 status

Since the present data of HCRP1 suggest a crucial role of HCRP1 for endosomal RTK- degradation, it might have the potential of influencing the prognostic impact of EGFR/HER2 overexpression on the survival of patients. Thus a tissue microarray (TMA) analysis was undertaken to test this hypothesis. Median follow-up for patients with malignant tumors was 40.0 months (range 0.4-168.7 months), and 38 patients (30.4%) had already died. The TMAs were immunostained for EGFR and HER2 expression, as these receptors represent 1) well-established markers for ovarian carcinogenesis and progression and are 2) subject to HCRP1-dependent receptor degradation. HER2 receptor was stained with the DAKO HercepTest and interpreted following the standard procedures for breast cancer diagnosis. The same interpretation procedure was used for the EGFR. Not unexpectedly, the expression of EGFR (p=0.015) as well as HER2 (p=0.003) significantly influenced overall patient survival, thus confirming previous results and those of others (Fig. 1) (Pils et al., 2007). Further, the TMAs were immunostained with the aforementioned HCRP1-specific antibody and the patient cohort divided into two subgroups (Median). Interestingly, HCRP1-low expressing tumours exhibited a statistically significant impact of EGFR and HER2 expression levels on overall survival which was lost in the corresponding HCRP1-high expressing tumors. This observation suggested a significant clinical influence of receptor decay mechanisms on the physiologic relevance of pathologic receptor expression. Thus, EGFR or HER2 overexpression led to more aggressive cancers, especially under conditions of low HCRP1 expression. The latter reflects the notion of a disproportionately high number of activated RTK, which are unable to be counterbalanced by reduced HCRP1-mediated degradation.

### Knock-down of HCRP1 in SK-OV-3 and MDAH cell lines:

In order to investigate the role of HCRP1 in the process of tyrosine kinase receptor degradation HCRP1 was knocked down and over-expressed in ovarian cancer cell lines, namely SKOV-3 and MDAH-2774 (Fig. 3). They were selected because of their high (SKOV-3) and low (MDAH-2774) expression levels of Her2Neu, while having equal amounts of EGFR (Fig. 2). Since activated EGFR and Her2Neu degradation is affected by the expression level of HCRP1, SK-OV-3 and MDAH cell lines provide a good basis to investigate the functional dynamics between these receptors and HCRP1.The knock-down of HCRP1 was evaluated on mRNA level by quantitative RT-PCR and on protein level by western blotting, leading to comparable results. For each cell line, the clone with the best knock-down capacity was selected for further experiments. Quantitative RT-PCR yielded a knock-down to 39% (MDAH) and 36% (SK-OV-3), respectively.

### HCRP1 is involved in the degradation process of the activated EGFR:

An inverse correlation between HCRP1 and the protein level of the activated EGFR and HER2 receptors was found. Expression levels of total and activated EGFR protein was quantified in five different MDAH clones and one SK-OV-3 clone with reduced HCRP1 expression. While the total receptor amount was independent of HCRP1 expression, pEGFR was found to be highly increased in HCRP1 silenced clones, suggesting that the degradation process of the activated receptor is depleted in these clones (Fig. 4). This tight correlation was confirmed by statistical analysis (Pearsons C. =0915, p<0,011).

The intracellular compartment harboring aberrantly retained pEGFR protein was further defined: Therefore, ovary cells were grown on coverslips and probed with an anti-EGFR antibody. In control cell lines, pEGFR was located mainly at the cell membrane (SK-OV-3) or was hardly detectable. In contrast, in both HCRP1-deficient cell lines pEGFR was detected at much higher levels in the cytoplasm (Fig. 5).

To gain further insight into the process behind these observations, the dynamics of pEGFR degradation was studied in wildtype and HCRP1-deficient MDAH and SK-OV-3 cells. Upon incubation in serum-free media, cells were stimulated with 100 ng/ml EGF for 15min to achieve maximal receptor activation, followed by re-incubation in serum-free media. At defined time points, cells were harvested, proteins prepared, and pEGFR expression was determined by Western blotting. pEGFR degradation was significantly impaired in both HCRP-1 deficient cell lines compared with control cells. Interestingly, the EGFR degradation process of the SK-OV-3 cell line was even stronger inhibited compared to the MDAH cell line. (Fig. 6).

### HCRP1 knock-down leads to Cetuximab resistance in SK-OV-3 cells:

Proliferation of HCRP1 silenced SKOV-3 cells was monitored by subsequent seeding and quantifying the cell number. The results are depicted in Fig. 5 as 1/doubling time. Non-induced cells (HCRP1+), as well as induced cells (HCRP1-) had essentially the same doubling time (25.5 and 24.9 hours respectively). Thus, proliferation of SKOV-3 cells was not affected by HCRP1 levels. Interestingly, upon incubation with Cetuximab, an antagonistic antibody against EGFR, only the proliferation of HCRP1+ cells decreased significantly (doubling time 32.7 hours), whereas HCRP1- cells were not affected (26.3 hours), suggesting that the knock down of HCRP1 triggers SKOV-3 cells to be resistant against Cetuximab treatment. To investigate, if this effect is restricted to the Cetuximab specific inactivation mechanism, a further proliferation assay was performed in presence of Lapatinib, a small molecule targeted against tyrosine kinase (TK) domain of the receptor. The proliferation of SKOV-3 cells was strongly depressed upon Lapatinib treatment (doubling time 37.3 hours), but was unaffected by HCRP1 expression (Fig. 7).

To confirm the effect of HCRP1 on Cetuximab treated SKOV3 cells by a second independent method, a CellTiter-Blue assay was performed. The impact of HCRP1 expression on the proliferation was calculated as quotient of HCRP1+ and HCRP1- cells. A ratio of 1 would mean that no change of proliferation was detected between HCRP1+ and HCRP1- cells. Ratios below/beyond 1 come along with increased/decreased proliferation of HCRP1+ cells or vice versa for HCRP1- cells. HCRP1 knock down yielded no effect on proliferation in non-stimulated cells, whereas EGF concentrations of 5ng/ml or higher resulted in a slightly increased proliferation of HCRP1- cells (ratio=0.8-0.9). Upon incubation with Cetuximab the ratio declined significantly (0.68-0.78), what can be traced back to decreased proliferation of HCRP1+ cells, while HCRP1-cells were not affected (Fig. 8). Overexpression of HCRP1 in SKOV-3 cells had no effect on proliferation, no matter if cells were treated or not with Cetuximab or Lapatinib (data not shown). This may be traced back to limiting amounts of different ESCRT I subunits, so that the active complex can not be formed.

### Discussion:

HCRP1 silenced cells overcome the proliferation inhibitory effect of Cetuximab, but not of Lapatinib. This result is supported by several studies dealing with the properties of Cetuximab and Lapatinib in EGFR inactivation. By occupying the ligand binding site, Cetuximab triggers the receptor to be internalized and degraded. It was suggested that binding of Cetuximab leads to EGR activation by hyperphosphorylation of tyrosine 1173 prior to downregulation. In HCRP1 silenced cells treated with Cetuximab, the activated receptor may accumulate in the cytoplasm still giving a signal, since receptor degradation is depleted in HCRP1 silenced cells and Cetuximab inhibits the recycling process of the receptor. In contrast and in line with the results of the proliferation assay, Lapatinib directly anticipates receptor activation by targeting the ATP-binding site and thus interrupts downstream signalling independent of ESCRT mediated receptor down-regulation. Up to now, there has no rational criterium been available in the prior art for the decision between Cetuximab and Lapatinib in targeted therapies against EGFR overexpressing tumours, a dysregulation found in various cancers like breast, ovary, head and neck, prostate, bladder, kidney and lung (Yarden et al., Nature Reviews Molecular Cell Biology 2, 127-137, 2001). The results according to the present invention show that HCRP1 has the potential to serve as a molecular marker to decide, whether treatment with Cetuximab is indicated or not. Since HCRP1 is active as part of a protein complex (ESCRT-I), limiting expression (or mutations) of other members of the same protein complex also leads to similar effects concerning the inhibitory potential of Cetuximab. The whole complex comprises Vps23 (vacuolar protein sorting 23), Vps28, Mvb12 (multivesicular body sorting factor of 12 kD) and HCRP1. According, the invention as shown in the present example section for HCRP1 can also be performed by using the other complex proteins Vps23, Vps28 and Mvb12.

## Claims

1. : Method for determining the status of a tumour patient with respect to tumour treatment with an anti-Epidermal Growth Factor Receptor (EGFR-) antibody **characterised by** the following steps:
- determining the amount of mRNA or protein of endosomal sorting complex required for transport (ESCRT) selected from Hepatocellular Carcinoma Related Protein 1 (HCRP1), vacuolar protein sorting 23 (Vps23), vacuolar protein sorting 28 (Vps28) and multivesicular body sorting factor of 12 kD (Mvb12), in a tumour biopsy sample of a tumour patient;
- determining the status of the tumour patient as being eligible for the treatment with an anti-EGFR antibody if the ratio of the HCRP1, Vps23, Vps28 or Mvb12 amount as determined in the tumour biopsy sample and the HCRP1, Vps23, Vps28 or Mvb12 amount as determined in normal, non-tumour tissues is at least 0.8; or
- determining the status of the tumour patient as not being eligible for the treatment with an anti-EGFR antibody if the ratio of HCRP1, Vps23, Vps28 or Mvb12 amount determined in the tumour biopsy sample and the HCRP1, Vps23, Vps28 or Mvb12 amount in normal, non-tumour tissue is less than 0.8.

2. : A method for determining the likelihood of effectiveness of an anti-EGFR antibody treatment in a tumour patient comprising:
- determining the amount of HCRP1, Vps23, Vps28 or Mvb12 in a biopsy sample of a tumour patient;
- determining the likelihood of effectiveness of an EGFR targeting treatment with an anti-EGFR antibody if the HCRP1, Vps23, Vps28 or Mvb12 amount determined in the tumour biopsy sample is at least 80%, or equal to, or above the HCRP1, Vps23, Vps28 or Mvb12 amount in normal, non-tumour tissue.

3. : A method of treating a patient affected with or at risk for developing a tumour with an anti-EGFR antibody, comprising detecting the presence or absence of reduced HCRP1, Vps23, Vps28 or Mvb12 expression in a tumour biopsy of said patient, wherein the patient is administered an anti-EGFR antibody treatment if the absence of the reduced HCRP1, Vps23, Vps28 or Mvb12 expression is detected.

4. : A method to direct treatment of a patient with a tumour, wherein the HCRP1, Vps23, Vps28 or Mvb12 expression status of a tumour biopsy of the patient is determined, wherein the absence of a reduced HCRP1 expression directs treatment of the patient towards administration of an anti-EGFR antibody treatment.

5. : The use of a kit for determining the expression of HCRP1 in a tumour biopsy sample, comprising
- an antibody against HCRP1, Vps23, Vps28 or Mvb12, and
- detection means for the antibody against HCRP1, Vps23, Vps28 or Mvb12; or
- a primer against HCRP1, Vps23, Vps28 or Mvb12, and
- detection means for the amount of mRNA of HCRP1, Vps23, Vps28 or Mvb12 as determined by quantitative RT-PCR
for predicting the efficiency of an anti-EGFR antibody treatment of the tumour patient

6. : Method for determining the status of a tumour patient with respect to tumour treatment with an anti-Epidermal Growth Factor Receptor (EGFR-) antibody **characterised by** the following steps:
- determining the amount of Hepatocellular Carcinoma Related Protein 1 (HCRP1) mRNA or protein in a tumour biopsy sample of a tumour patient;
- determining the status of the tumour patient as being eligible for the treatment with an anti-EGFR antibody if the ratio of the HCRP1 amount as determined in the tumour biopsy sample and the HCRP1 amount as determined in normal, non-tumour tissues is at least 0.8; or
- determining the status of the tumour patient as not being eligible for the treatment with an anti-EGFR antibody if the ratio of HCRP1 amount determined in the tumour biopsy sample and the HCRP1 amount in normal, non-tumour tissue is less than 0.8. As reference for normal, non-tumor tissue may be a non-tumor section of the sample tissue taken from the same patient. A further reference may be the experimentally determined tissue specific expression of HCRP1. A further reference may be the expression levels as identified in a number of normal tissues. A further reference may be an absolute reference.

7. : Method according to claim 6 **characterised in that** the anti-EGFR antibody is Cetuximab, Matuzumab, Pertuzumab, Zalutumumab, Nimotuzumab or Panitumumab.

8. : Method according to claim 6 or 7, **characterised in that** the tumour patient has gastrointestinal cancer, prostate cancer, ovarian cancer, breast cancer, head and neck cancer, lung cancer, non-small cell lung cancer, cancer of the nervous system, kidney cancer, retinal cancer, skin cancer, stomach cancer, liver cancer, pancreatic cancer, genital-urinary cancer, prostate cancer, colorectal cancer, rectal cancer or bladder cancer.

9. : A method for determining the likelihood of effectiveness of an anti-EGFR antibody treatment in a tumor patient comprising:
determining the amount of Hepatocellular Carcinoma Related Protein 1 (HCRP1) in a biopsy sample of a tumour patient;
- determining the likelihood of effectiveness of an EGFR targeting treatment with an anti-EGFR antibody if the HCRP1 amount determined in the tumour biopsy sample is at least 80% of the HCRP1 amount in normal, non-tumour tissue.

10. : A method of treating a patient affected with or at risk for developing a tumour with an anti-EGFR antibody, comprising detecting the presence or absence of reduced HCRP1 expression in a tumour biopsy of said patient, wherein the patient is administered an anti-EGFR antibody treatment if the absence of the reduced HCRP1 expression is detected.

11. : A method to direct treatment of a patient with a tumor, wherein the HCRP1 expression status of a tumour biopsy of the patient is determined, wherein the absence of a reduced HCRP1 expression directs treatment of the patient towards administration of an anti-EGFR antibody treatment.

12. : A method according to any one of claims 9 to 11, **characterised in that** the tumour patient has gastrointestinal cancer, prostate cancer, ovarian cancer, breast cancer, head and neck cancer, lung cancer, non-small cell lung cancer, cancer of the nervous system, kidney cancer, retinal cancer, skin cancer, stomach cancer, liver cancer, pancreatic cancer, genital-urinary cancer, prostate cancer, colorectal cancer, rectal cancer or bladder cancer.

13. : The use of a kit for determining the expression of HCRP1 in a tumour biopsy sample, comprising
- an antibody against HCRP1, and
- detection means for the antibody against HCRP1 for predicting the efficiency of an anti-EGFR antibody treatment of the tumour patient.

14. : The use of a kit for determining the expression of HCRP1 in a tumour biopsy sample, comprising
- a primer against HCRP1, and
- detection means for the amount of mRNA of HCRP1 as determined by quantitative RT-PCR
for predicting the efficiency of an anti-EGFR antibody treatment of the tumour patient.

15. : Antibody against HCRP1, **characterised in that** it is raised against antigenic determinants of HCRP1 selected from the sequences MSPYASGGFPFLPPY, WLFPLTKSASSSAAG, or TSHTTAKPAAPSFGV.


SEQUENCE LISTING

<110>  Medizinische Universität Wien

<120>  Improvement of Tumour Treatment

<130>  R 52959

<160>  3

<170>  PatentIn version 3.4

<210>  1
<211>  15
<212>  PRT
<213>  human

<400>  1

Met Ser Pro Tyr Ala Ser Gly Gly Phe Pro Phe Leu Pro Pro Tyr
1               5                   10                  15


<210>  2
<211>  15
<212>  PRT
<213>  human

<400>  2

Trp Leu Phe Pro Leu Thr Lys Ser Ala Ser Ser Ser Ala Ala Gly
1               5                   10                  15


<210>  3
<211>  15
<212>  PRT
<213>  human

<400>  3

Thr Ser His Thr Thr Ala Lys Pro Ala Ala Pro Ser Phe Gly Val
1               5                   10                  15